(19) Europäisches Patentamt
European Patent Office
Office européen des brevets

(11) Veröffentlichungsnummer: **0 216 290 A2**

(12) **EUROPÄISCHE PATENTANMELDUNG**

(21) Anmeldenummer: 86112758.7

(51) Int. Cl.[4]: **A23K 1/16**, C07D 333/26

(22) Anmeldetag: 16.09.86

(30) Priorität: 27.09.85 DE 3534421

(43) Veröffentlichungstag der Anmeldung: 01.04.87 Patentblatt 87/14

(84) Benannte Vertragsstaaten: AT BE CH DE FR GB IT LI NL SE

(71) Anmelder: **BAYER AG**
**Konzernverwaltung RP Patentabteilung**
**D-5090 Leverkusen 1 Bayerwerk(DE)**

(72) Erfinder: **Hallenbach, Werner, Dr.**
**Kleiststrasse 10**
**D-4018 Langenfeld(DE)**
Erfinder: **Lindel, Hans, Dr.**
**Carl-Duisberg-Strasse 321**
**D-5090 Leverkusen 1(DE)**
Erfinder: **Berschauer, Friedrich, Dr.**
**Claudiusweg 9**
**D-5600 Wuppertal 1(DE)**
Erfinder: **Scheer, Martin, Dr.**
**Herberts-Katernberg 7**
**D-5600 Wuppertal 1(DE)**
Erfinder: **de Jong, Anno, Dr.**
**Stockmannsmühle 46**
**D-5600 Wuppertal 1(DE)**

(54) **Verwendung von Thienylaminen als leistungsfördernde Mittel, neues Thienylamin.**

(57) Die vorliegende Erfindung betrifft leistungsfördernde Mittel für Tiere, die durch einen Gehalt an substituierten Thienylaminen der Formel I

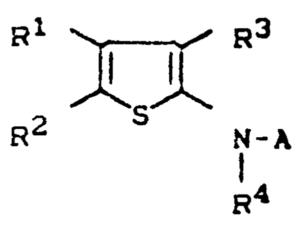

I

in welcher

$R^1$, $R^2$, $R^3$, $R^4$, A die in der Beschreibung angegebene Bedeutung haben,

gekennzeichnet sind.

EP 0 216 290 A2

## Verwendung von Thienylaminen als leistungsfördernde Mittel, neues Thienylamin

Die vorliegende Erfindung betrifft die Verwendung von teilweise bekannten substituierten Thienylaminen als leistungsfördernde Mittel bei Tieren.

Thienylamine sind bereits bekannt geworden. Sie finden Verwendung als Pflanzenschutzmittel, Zwischenprodukte zur Farbstoffherstellung und Pharmazeutika (vgl. DE-OS 2 122 636, EP-OS 4 931, US-P 4 317 915, 4 447 624).

Es ist jedoch nichts über ihren Einsatz als leistungsfördernde Mittel bei Tieren bekannt geworden.

1. Es wurde gefunden, daß substituierte Thienylamine der Formel I

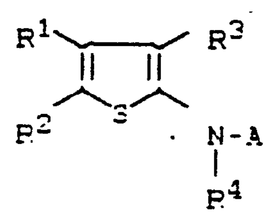

I

in welcher

A für den Rest I a steht

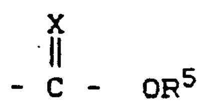

Ia

X für O oder S steht,

$R^1$ für Wasserstoff, Halogen, Nitro, CN, Alkoxy, Alkylthio, Halogenalkoxy, Halogenalkylthio, gegebenenfalls substituierte Reste aus der Gruppe Alkyl, Acyl, Aroyl, Aryl steht,

$R^2$ für Wasserstoff, Halogen, Nitro, CN, Alkoxy, Alkylthio, Halogenalkoxy, Halogenalkylthio, gegebenenfalls substituierte Reste aus der Gruppe Acyl, Aroyl, Alkyl, Aryl steht,

$R^1$ und $R^2$ gemeinsam mit den angrenzenden C-Atomen für einen gegebenenfalls substituierten gesättigten oder ungesättigten carbocyclischen oder heterocyclischen Ring stehen, der gegebenenfalls eine Carbonylfunktion tragen kann,

$R^3$ für die Reste Cn, $COR^6$, $COOR^7$, $CONR^8R^9$ steht,

$R^4$ für Wasserstoff oder Alkyl steht,

$R^5$ für gegebenenfalls substituiertes Alkyl, Cycloalkyl, Alkenyl, gegebenenfalls substituiertes Aryl steht,

$R^6$ für gegebenenfalls substituiertes Alkyl oder gegebenenfalls substituiertes Aryl steht,

$R^7$ für Wasserstoff, gegebenenfalls substituiertes Alkyl, Cycloalkyl, Alkenyl, gegebenenfalls substituiertes Aryl steht,

$R^8$ für Alkyl oder Cycloalkyl steht,

$R^9$ für gegebenenfalls substituiertes Alkyl steht,

hervorragende leistungsfördernde Wirkung bei Tieren besitzen.

Die substituierten Thienylamine der Formel I sind teilweise bekannt. Soweit sie neu sind, können sie nach an sich bekannten Methoden hergestellt werden.

2-Aminomethoxycarbonyl-3-carboethoxy-4-ethyl-5-methyl thiophen ist neu.

Es war völlig überraschend, daß die substituierten Thienylamine der Formel I leistungsfördernde Eigenschaften bei Tieren aufweisen. Es gab aus dem Stand der Technik keinerlei Hinweis auf diese neue Verwendung der teilweise bekannten substituierten Thienylamine der Formel I.

Bevorzugt sind substituierte Thienylamine der Formel I in welcher

A für den Rest Ia steht,

X für O oder S steht,

$R^1$ für Wasserstoff, Halogen, Nitro, CN, $C_{1-4}$-Alkoxy, $C_{1-4}$-Alkylthio, gegebenenfalls substituiertes $C_{1-6}$-Acyl, gegebenenfalls substituiertes Aroyl, insbesondere Benzoyl, für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$-alkylamino, Arylamino, insbesondere Phenylamino substituiertes $C_{1-6}$-Alkyl sowie für Phenyl steht, wobei die Phenylreste gegebenenfalls einen oder mehrere der folgenden Substituenten tragen: Halogen, $C_{1-4}$-Alkyl, CN, C $_{1-4}$-Alkoxy, $C_{1-4}$-Alkylthio, Phenyl, Phenoxy, Phenylthio, Amino, $C_{1-4}$-Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$-Halogenalkylthio, Methylendioxy oder Ethylendioxy, die gegebenenfalls halogensubstituiert sind, Acyl,

$R^2$ für die bei $R^1$ aufgeführten Reste steht.

$R^1$ und $R^2$ gemeinsam mit den angrenzenden beiden C-Atomen für gesättigte oder ungesättigte carbocyclische Reste mit 5-8 Ringgliedern steht, die gegebenenfalls durch OH, $C_{1-4}$-Alkyl, Halogen, Nitro, CN, $C_{1-4}$-Alkoxy, C $_{1-4}$-Alkylthio, Phenyl, Phenoxy, Phenylthio, Amino, $C_{1-4}$ -Alkylamino, $C_{1-4}$-Dialkylamino, $C_{1-4}$-Halogenalkyl, $C_{1-4}$-Halogenalkoxy, $C_{1-4}$-Halogenalkylthio, $C_{1-4}$-Alkoxyalkyl substituiert sind und eines der Ringglieder, die nicht an den Thiophenring gebunden sind, eine Carbonylfunktion (C = O) tragen kann; für den Fall, daß $R^1$ und $R^2$ mit den angrenzenden C-Atomen einen heterocyclischen Ring bilden, hat dieser 5 -6 Ringglieder und trägt O, S oder N als Heteroatome.

$R^3$ für die Rest CN, $COR^6$, $COOR^7$, $CONR^8R^9$ steht,

$R^4$ für Wasserstoff oder $C_{1-4}$-Alkyl steht,

$R^5$ für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$-alkylamino substituiertes $C_{1-6}$-Alkyl, $C_{3-8}$-Cycloalkyl, C $_{2-6}$-Alkenyl ferner für Phenyl oder Naphthyl steht, wobei die Phenylreste gegebenenfalls einen oder mehrere der folgenden Substituenten tragen: Halogen, $C_{1-4}$-Alkyl, CN, $C_{1-4}$-Alkoxy, C $_{1-4}$-Alkylthio, Phenyl, Phenoxy, Phenylthio, Amino, $C_{1-4}$ -Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$-Halogenalkylthio, Methylendioxy oder Ethylendioxy, die gegebenenfalls halogensubstituiert sind,

$R^6$ für gegebenenfalls durch Halogen, $C_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$ -alkylamino substituiertes $C_{1-6}$-Alkyl, ferner für Phenyl oder Naphthyl steht, wobei die Phenylreste gegebenenfalls einen oder mehrere der folgenden Substituenten tragen: Halogen, $C_{1-4}$-Alkyl, CN, $C_{1-4}$-Alkoxy, $C_{1-4}$-Alkylthio, Phenyl, Phenoxy, Phenylthio, Amino, $C_{1-4}$-Alkylamino, Di-$C_{1-4}$ -alkylamino, $C_{1-4}$-Alkoxyalkyl, $C_{1-4}$-Halogenalkyl, $C_{1-4}$-Halogenalkoxy, $C_{1-4}$ -Halogenalkylthio, Methylendioxy oder Ethylendioxy, die gegebenenfalls halogensubstituiert sind,

$R^7$ für gegebenenfalls durch Halogen, C $_{1-4}$-Alkoxy, $C_{1-4}$-Alkylthio, Aryl, insbesondere Phenyl, Aryloxy, insbesondere Phenoxy, Arylthio, insbesondere Phenylthio, Amino, $C_{1-4}$-Alkylamino, Di-$C_{1-4}$-alkylamino substituiertes $C_{1-6}$-Alkyl, $C_{3-8}$-Cycloalkyl, C $_{2-6}$-Alkenyl ferner für Phenyl oder Naphthyl steht, wobei die Phenylreste gegebenenfalls einen oder mehrere der folgenden Substituenten tragen: Halogen, $C_{1-4}$-Alkyl, CN, $C_{1-4}$-Alkoxy, C $_{1-4}$-Alkylthio, Phenyl, Phenoxy, Phenylthio, Amino, $C_{1-4}$ -Alkylamino, Di-$C_{1-4}$-alkylamino, $C_{1-4}$-Alkoxyalkyl, $C_{1-4}$-Halogenalkyl, $C_{1-4}$-Halogenalkoxy, $C_{1-4}$-Halogenalkylthio, Methylendioxy oder Ethylendioxy, die gegebenenfalls halogensubstituiert sind,

$R^8$ für C $_{1-4}$-Alkyl, $C_{3-8}$-Cycloalkyl steht,

$R^9$ für $C_{1-4}$-Alkyl steht.

Besonders bevorzugt sind Verbindungen der Formel I, in welcher

A für den Rest Ia in 2-Stellung des Thiophenrings steht,

X für O steht,

$R^1$ für Wasserstoff, $C_{1-6}$-Alkyl, das gegebenenfalls durch Fluor, Chlor oder Brom substituiert ist, Phenyl, das gegebenenfalls durch $C_{1-4}$-Alkyl, Halogen, $C_{1-4}$-Halogenalkyl, insbesondere Trifluormethyl, $C_{1-4}$-Halogenalkoxy, insbesondere Trifluormethoxy substituiert ist, für Nitro, Acyl, insbesondere Acetyl, steht.

$R^2$ für die bei $R^1$ angegebenen Reste steht,

$R^1$ und $R^2$ gemeinsam für $-(CH_2)_{3-6}$ -stehen

$R^3$ in 3-Stellung des Thiophenrings für die Reste CN, $COR^6$, $COOR^7$, $CONR^8R^9$ steht,

$R^4$ für Wasserstoff steht,

$R^5$ für $C_{1-6}$-Alkyl, $C_{1-4}$-Alkylthio-$C_{1-4}$-alkyl, Cycloalkyl mit bis zu 8 C-Atomen, $C_{2-4}$-Alkenyl, Phenyl, das gegebenenfalls durch $C_{1-4}$-Alkyl, $C_{1-4}$-Halogenalkyl, $C_{1-4}$-Alkoxy, Halogen, insbesondere Chlor, Nitro, substituiert ist, oder Naphthyl steht.

$R^6$ für $C_{1-4}$-Alkyl, insbesondere Methyl und Ethyl, steht,

$R^7$ für $C_{1-4}$-Alkyl, insbesondere Methyl, Ethyl, n-, i-Propyl, n-, t-Butyl, $C_{2-4}$-Alkenyl, insbesondere Allyl, sowie für Phenyl steht,

$R^8$ für $C_{1-4}$-Alkyl steht,

$R^9$ für $C_{1-4}$-Alkyl, insbesondere Methyl, Ethyl steht.

Insbesondere seien Verbindungen der Formel I genannt, in welcher

A in 2-Stellung des Thiophenrings für den Rest der Formel Ia steht,

$R^1$ für Wasserstoff, $C_{1-5}$-Alkyl, insbesondere Methyl, Ethyl, Isopropyl, t-Butyl, n-Pentyl, Acetyl, Phenyl, Nitro steht,

$R^2$ für die bei $R^1$ angeführten Reste steht,

$R^1$ und $R^2$ gemeinsam für einen an den Thiophenring ankondensierten Cyclopentan-, Cyclohexan-, Cycloheptan-, Cyclooctanring stehen,

$R^3$ in 3-Stellung des Thiophenrings für die Reste CN, $COR^6$, $CONR^8R^9$, $COOR^7$ steht,

$R^4$ für Wasserstoff steht,

$R^5$ für $C_{1-6}$-Alkyl, Cycloalkyl mit bis zu 6 C-Atomen, Phenyl, das gegebenenfalls durch Halogen, insbesondere Chlor, Nitro, Methyl, i-Propyl, Methoxy, Trifluormethyl substituiert ist, sowie für Naphthyl steht,

$R^7$ für $C_{1-4}$-Alkyl, insbesondere Methyl, Ethyl, n-, i-Propyl, n-, t-Butyl, sowie für Phenyl steht,

$R^8$ für Methyl steht,

$R^9$ für Methyl steht.

Im einzelnen seien neben den in den Beispielen genannten die folgenden Verbindungen genannt:

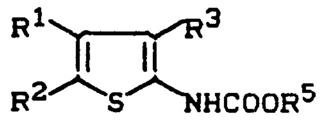

| $R^1+R^2$ | $R^3$ | $R^5$ |
|---|---|---|
| $-(CH_2)_3-$ | CN | $i-C_3H_7$ |
| " | " | $i-C_4H_9$ |
| " | " | $s-C_4H_9$ |
| " | " | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |
| " | " | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |
| " | $COOC_2H_5$ | $i-C_3H_7$ |
| " | " | $i-C_4H_9$ |
| " | " | $s-C_4H_9$ |

| $R^1$+$R^2$ | $R^3$ | $R^5$ |
|---|---|---|
| $-(CH_2)_3-$ | $COOC_2H_5$ | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |
| " | " | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |
| $-(CH_2)_4-$ | " | $i-C_3H_7$ |
| " | " | $i-C_4H_9$ |
| " | " | $s-C_4H_9$ |
| " | " | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |
| " | " | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |

| $R^1+R^2$ | $R^3$ | $R^5$ |
|---|---|---|
| $-(CH_2)_4-$ | CN | $i-C_3H_7$ |
| " | " | $i-C_4H_9$ |
| " | " | $s-C_4H_9$ |
| " | " | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |
| " | " | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |
| $-(CH_2)_5-$ | " | $i-C_3H_7$ |
| " | " | $i-C_4H_9$ |
| " | " | $s-C_4H_9$ |
| " | " | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |

| $R^1+R^2$ | $R^3$ | $R^5$ |
|---|---|---|
| $-(CH_2)_5-$ | CN | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |
| " | $COOC_2H_5$ | $i-C_3H_7$ |
| " | " | $i-C_4H_9$ |
| " | " | $s-C_4H_9$ |
| " | " | $t-C_4H_9$ |
| " | " | $c-C_5H_{11}$ |
| " | " | $c-C_6H_{13}$ |
| " | " | $C_6H_5$ |
| " | " | $4-CH_3-O-C_6H_4$ |
| " | " | $2-CH_3-O-C_6H_4$ |
| " | " | $2-CH_3-C_6H_4$ |
| " | " | $3-CH_3-C_6H_4$ |
| " | " | $(2,3-CH_3)C_6H_3$ |
| " | " | $(3,4-CH_3)C_6H_3$ |
| " | " | $(2,6-CH_3)C_6H_3$ |
| " | " | $2-i-C_3H_7-C_6H_4$ |
| " | " | $(2,3,5-CH_3)C_6H_2$ |
| " | " | $1-C_{10}H_7$ |
| " | " | $2-C_{10}H_7$ |

Die Thienylamine oder -carbamate der Formel I sind teilweise bekannt. Sie lassen sich analog zu bekannten Verfahren herstellen (DE-OS 2 122 636, US-P 4 317 915).

Die Wirkstoffe werden als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier-und Hobbytieren verwendet.

Zu den Nutz-und Zuchttieren zählen Säugetiere wie z.B. Rinder, Schweine, Pferde, Schafe, Ziegen, Kaninchen, Hasen, Damwild, Pelztiere wie Nerze, Chinchilla, Geflügel wie z.B. Hühner, Puten, Gänse, Enten, Tauben, Fische wie z.B. Karpfen, Forellen, Lachse, Aale, Schleien, Hechte, Reptilien wie z.B. Schlangen und Krokodile.

Zu den Zier-und Hobbytieren zählen Säugetiere wie Hunde und Katzen, Vögel wie Papageien, Kanarienvögel, Fische wie Zier-und Aquarienfische z.B. Goldfische.

Die Wirkstoffe werden unabhängig vom Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstumsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Die Verabreichung erfolgt oral oder parenteral in dafür geeigneten Formulierungen oder in reiner Form. Orale Formulierungen sind Pulver, Tabletten, Granulate, Doenche, Boli sowie Futtermittel, Prämixe für Futtermittel, Formulierungen zur Verabreichung über Trinkwasser.

Die oralen Formulierungen enthalten den Wirkstoff in Konzentrationen von 0,01 ppm -100 %, bevorzugt von 0,01 ppm -1 %.

Parenterale Formulierungen sind Injektionen in Form von Lösungen, Emulsionen und Suspensionen, sowie Implantate.

Die Wirkstoffe können in den Formulierungen allein oder in Mischung mit anderen Wirkstoffen, Mineralsalzen, Spurnelementen, Vitaminen, Eiweißstoffen, Farbstoffen, Fetten oder Geschmacksstoffen vorliegen.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthällt:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten:

600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_2$ x $H_2O$, 140 mg Zn $SO_4$ x 7 $H_2O$, 100 mg $FeSO_4$ x 7 $H_2O$ und 20 mg Cu $SO_4$ x 5 $H_2O$.

2,5 g Wirkstoff-Prämix enthalten z.B. 10 mg Wirkstoff, 1 g DL-Methionin, Rest Sojabohnenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix - (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt.

Es werden die aus der Tabelle ersichtlichen Ergebnisse erhalten:

Tabelle1: Ratten-Fütterungsversuch

| Wirkstoff | Dosis | Gewichtszunahme |
|---|---|---|
| Kontrolle, ohne Wirkstoff | | 100 |
| Bsp. 1 | 25 ppm | 119 |
| Bsp. 19 | " | 115 |
| Bsp. 20 | " | 109 |
| Bsp. 31 | " | 109 |
| Bsp. 56 | " | 116 |

Herstellungsbeispiele

Beispiel 1

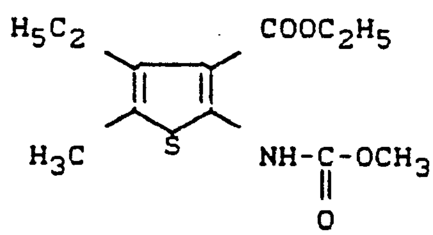

2-Aminomethoxycarbonyl-3-carbethoxy-4-ethyl-5-methylthiophen

5 g (0,021 mol) 3-Carbethoxy-4-ethyl-2-isocyanato-5-methyl-thiophen wurden zu 40 ml trockenem Methanol gegeben und eine Stunde unter Rückfluß erhitzt. Danach wurde überschüssiges Methanol im Vakuum abdestilliert und der Rückstand im Kugelrohr an der Ölpumpe destilliert. Ausbeute 3,7 g (65 %), Siedepunkt 156° C (1 Pa), IR: 3300, 1730, 1660.

Beispiel 2

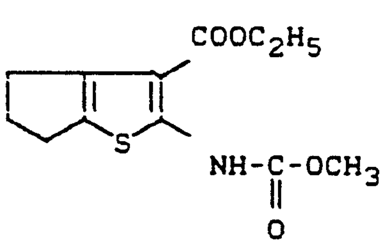

2-Methoxycarbonylamino-3-ethoxycarbonyl-4,5-trimethylenthiophen

6,33 g (30 mmol) 2-Amino-3-ethoxycarbonyl-4,5-trimethylenthiophen und 2,37 g (30 mmol) Pyridin werden in 50 ml trockenem Chloroform mit einer Lösung von 3,1 g (33 mmol) Chlorameisensäuremethylester in 20 ml trockenem Chloroform versetzt. Nach einer Stunde wird 3 x mit je 30 ml Wasser, 1 x mit 30 ml 10 %iger Salzsäure und noch 2 x mit je 30 ml Wasser gewaschen, über $Na_2SO_4$ getrocknet, eingedampft und aus Ethanol umkristallisiert.

Beigefarbene Kristalle, Fp. 102° C, Ausbeute 6 g - (75 %).

Beispiel 3

2-Phenoxycarbonylamino-3-carbethoxy-4-ethyl-5-methyl-thiophen

Zu einer Lösung von 5 g (0,021 mol) 3-Carbethoxy-2-isocyanato-4-ethyl-5-methyl-thiophen in 40 ml trockenem Toluol werden 2,2 g ( 0,023 mol) Phenol gegeben und das Ganze eine Stunde auf 60° C erwärmt. Danach wird abgekühlt und die organische Phase nacheinander mit je 100 ml 5%iger $H_2SO_4$, Wasser und gesättigter $NaHCO_3$-Lösung gewaschen. Das Toluol wird im Vakuum abdestilliert und der Rückstand aus verdünntem wässrigen Ethanol umkristallisiert. Ausbeute: 4,2 g (60 % der Theorie), Schmelzpunkt: 103° C.

Analog wurden die folgenden Verbindungen erhalten:

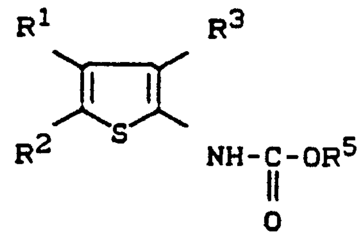

| Bsp. Nr. | $R^2$ | $R^1$ | $R^3$ | $R^5$ | Fp. [°C] |
|---|---|---|---|---|---|
| 4 | H | $CH_3$ | $COOC_2H_5$ | $i-C_3H_7$ | 77 |
| 5 | " | " | " | $s-C_4H_9$ | 51 |
| 6 | " | " | " | $t-C_4H_9$ | 74 |
| 7 | " | " | " | $i-C_4H_9$ | 51 |
| 8 | " | " | " | $c-C_5H_{11}$ | 50 |
| 9 | " | " | " | $C_6H_5$ | 119 |
| 10 | " | " | " | $c-C_6H_{13}$ | 87 |
| 11 | " | " | " | $(4-CH_3O)C_6H_4$ | 104-5 |
| 12 | " | " | " | $(2-CH_3O)C_6H_4$ | 135 |
| 13 | $n-C_5H_{11}$ | H | — | $i-C_3H_7$ | Öl |
| 14 | " | " | " | $i-C_4H_9$ | Öl |
| 15 | " | " | " | $s-C_4H_9$ | Öl |
| 16 | " | " | " | $t-C_4H_9$ | Öl |
| 17 | " | " | " | $c-C_5H_{11}$ | Öl |
| 18 | " | " | " | $C_6H_5$ | Öl |
| 19 | " | " | " | $c-C_6H_{13}$ | Öl |
| 20 | " | " | " | $2-CH_3C_6H_4$ | Öl |
| 21 | " | " | " | $3-CH_3C_6H_4$ | Öl |
| 22 | " | " | " | $4-CH_3C_6H_4$ | Öl |
| 23 | " | " | " | $4-CH_3OC_6H_4$ | 64-5 |
| 24 | " | " | " | $2-CH_3OC_6H_4$ | Öl |
| 25 | " | " | " | $2,3-(CH_3)_2-C_6H_3$ | Öl |
| 26 | " | " | " | $3,4-(CH_3)_2-C_6H_3$ | Öl |
| 27 | " | " | " | $2,6-(CH_3)_2-C_6H_3$ | Öl |
| 28 | " | " | " | $2-(i-C_3H_7)-C_6H_4$ | Öl |
| 29 | " | " | " | $2,3,5-(CH_3)_3-C_6H_2$ | 43 |

| Bsp. Nr. | $R^2$ | $R^1$ | $R^3$ | $R^5$ | Fp oder Kp |
|---|---|---|---|---|---|
| 30 | " | " | " | $1-C_{10}H_7$ | Fp. 79° C |
| 31 | $-(CH_2)_4-$ | | $COOCH_3$ | $CH_3$ | Fp. 111° C |
| 32 | $-(CH_2)_5-$ | | $COOC_2H_5$ | $CH_3$ | Fp. 78° C |
| 33 | " | | CN | $CH_3$ | Fp. 129° C |
| 34 | $-CH_3$ | -Et | $COOC_2H_5$ | $i-C_3H_7$ | Kp 162° C 20 Pa |
| 35 | " | " | $CO_2C_2H_5$ | $-CH-CH_3$<br>$\vert$<br>$CH_2-CH_3$ | Kp 215° C 30 Pa |
| 36 | " | " | " | $2-CH_3OC_6H_4$ | Fp 101° C |
| 37 | " | H | " | $CH_3$ | Fp 52° C |
| 38 | " | " | " | $i-C_3H_7$ | Kp 122° C 10 Pa |
| 39 | " | " | " | $2-CH_3OC_6H_4$ | Fp 118° C |
| 40 | " | Et | " | $4-CH_3OC_6H_4$ | Fp 76° C |
| 41 | " | H | " | $4-CH_3OC_6H_4$ | Fp 99° C |
| 42 | " | " | " | $t-C_4H_9$ | Kp 144° C 30 Pa |
| 43 | " | " | " | $s-C_4H_9$ | Kp 143° C 30 Pa |
| 44 | " | " | " | $C_6H_5$ | Fp 87° C |
| 45 | $C_2H_5$ | $CH_3$ | " | $C_6H_5$ | Fp 61° C |
| 46 | " | " | " | $4-CH_3OC_6H_4$ | Fp 55° C |
| 47 | $i-C_3H_7$ | H | " | $i-C_3H_7$ | Kp 143° C 10 Pa |
| 48 | " | " | " | $C_6H_5$ | Fp 78° C |
| 49 | H | " | " | $CH_3$ | Fp 80° C |
| 50 | " | " | " | $i-C_3H_7$ | Fp 37° C |
| 51 | $C_2H_5$ | " | " | $CH_3$ | Kp 158° C 1 Pa |
| 52 | " | " | " | $s-C_4H_9$ | Kp 147° C 5 Pa |
| 53 | " | " | " | $i-C_3H_7$ | Kp 142° C 5 Pa |
| 54 | " | " | " | $4-CH_3OC_6H_4$ | Fp 67° C |
| 55 | " | " | " | $t-C_4H_9$ | Kp 161° C 5 Pa |
| 56 | " | " | " | $C_6H_5$ | Fp 56° C |
| 57 | " | " | " | $2-CH_3OC_6H_5$ | Fp 78° C |
| 58 | $i-C_3H_7$ | " | " | $t-C_4H_9$ | Kp 150° C 3 Pa |
| 59 | " | " | " | $4-CH_3OC_6H_4$ | Fp 108° C |
| 60 | " | " | " | $2-CH_3OC_6H_4$ | Fp 137° C |

| Bsp. Nr. | $R^2$ | $R^1$ | $R^3$ | $R^5$ | Fp oder Kp |
|---|---|---|---|---|---|
| 61 | $i-C_3H_7$ | H | $CO_2C_2H_5$ | $S-C_4H_9$ | Kp 149° C 3 Pa |
| 62 | $CH_3$ | " | " | $2-CH_3C_6H_4$ | Fp 76° C |
| 63 | $i-C_3H_7$ | " | " | " | Fp 65° C |
| 64 | $C_2H_5$ | " | " | " | Fp 67° C |
| 65 | $CH_3$ | " | " | $4-CH_3C_6H_4$ | Fp 78° C |
| 66 | $i-C_3H_7$ | " | " | " | Fp 88° C |
| 67 | $C_2H_5$ | " | " | " | Fp 82° C |

**Ansprüche**

1. Verwendung von substituierten Thienylaminen der Formel I

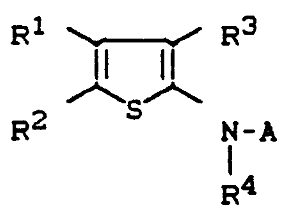

I

in welcher

A für den Rest Ia steht

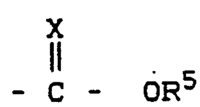

Ia

X für O oder S steht,

$R^1$ für Wasserstoff, Halogen, Nitro, CN, Alkoxy, Alkylthio, Halogenalkoxy, Halogenalkylthio, gegebenenfalls substituierte Reste aus der Gruppe Alkyl, Acyl, Aroyl, Aryl steht,

$R^2$ für Wasserstoff, Halogen, Nitro, CN, Alkoxy, Alkylthio, Halogenalkoxy, Halogenalkylthio, gegebenenfalls substituierte Reste aus der Gruppe Acyl, Aroyl, Alkyl, Aryl steht,

$R^1$ und $R^2$ gemeinsam mit den angrenzenden C-Atomen für einen gegebenenfalls substituierten gesättigten oder ungesättigten carbocyclischen oder heterocylischen Ring stehen, der gegebenenfalls eine Carbonylfunktion tragen kann,

$R^3$ für die Reste CN, $COR^6$, $COOR^7$, $CONR^8R^9$ steht,

$R^4$ für Wasserstoff oder Alkyl steht,

$R^5$ für gegebenenfalls substituiertes Alkyl, Cycloalkyl, Alkenyl, gegebenenfalls substituiertes Aryl steht,

$R^6$ für gegebenenfalls substituiertes Alkyl, oder

gegebenenfalls substituiertes Aryl steht,

$R^7$ für Wasserstoff, gegebenenfalls substituiertes Alkyl,Cycloalkyl, Alkenyl, gegebenenfalls substituiertes Aryl steht,

$R^8$ für Alkyl oder Cycloalkyl steht,

$R^9$ für gegebenenfalls substituiertes Alkyl steht,

als leistungsfördernde Mittel für Tiere.

2. Mittel zur Leistungsförderung von Tieren gekennzeichnet durch einen Gehalt an substituierten Thienylaminen der Formel I gemäß Anspruch 1.

3. Tierfutter, Trinkwasser für Tiere, Zusätze für Tierfutter und Trinkwasser für Tiere gekennzeichnet durch einen Gehalt an substituierten Thienylaminen der Formel I gemäß Anspruch 1.

4. Verwendung von substituierten Thienylaminen der Formel I gemäß Anspruch 1 zur Leistungsförderung von Tieren.

5. Verfahren zur Herstellung von Mitteln zur Leistungsförderung von Tieren, dadurch gekennzeichnet, daß man substituierte Thienylamine der Formel I gemäß Anspruch 1 mit Streck-und/oder Verdünnungsmitteln vermischt.

6. Verfahren zur Herstellung von Tierfutter, Trinkwasser für Tiere oder Zusätze für Tierfutter und Trinkwasser für Tiere, dadurch gekennzeichnet, daß man substituierte Thienylamine der Formel I gemäß Anspruch 1 mit Futtermitteln oder Trinkwasser und gegebenenfalls weiteren Hilfstoffen vermischt.

7. 2-Aminomethoxycarbonyl-3-carboethoxy-4-ethyl-5-methyl -thiophen.